# EUROPEAN PATENT APPLICATION

(11) **EP 4 763 213 A1**
(43) Date of publication of application: **24.06.2026**
(21) Application number: 24222469.9
(22) Date of filing: 20.12.2024
(51) Int. Cl.: A61K 35/747, A61P 1/00

(54) **COMPOSITION INCLUDING LACTOBACILLUS ACIDOPHILUS TW01 FOR USE IN ALLEVIATING COLITIS**

(71) Applicant: I Eating Light Ltd., Nantou City, Nantou County 540008 (TW)
(72) Inventor: CHIANG, Hsin-Hua, 814 KAOHSIUNG CITY (TW); LIOU, Bo-Jyun, 814 KAOHSIUNG CITY (TW)
(74) Representative: Regimbeau

(57) **Abstract**

A composition for use in alleviating colitis includes *Lactobacillus acidophilus* TW01 which is deposited at the Leibniz Institute DSMZ-German Collection of Microorganisms and Cell Cultures GmbH under an accession number DSM 33990 in accordance with the Budapest Treaty.

## Description

The present disclosure relates to a composition including *Lactobacillus acidophilus* TW01 for use in alleviating colitis.

Colitis is a form of inflammatory bowel disease (IBD) that may result from various factors such as diet, infections, stress, genetics, medications, gut microbiota imbalances, and immune system disorders. Patients with colitis often experience symptoms such as abdominal pain, diarrhea, nausea, vomiting, and fever. In severe cases, an increased risk of developing colon cancer may occur.

Current clinical treatments for colitis includes: (i) surgical intervention, such as removal of colon or rectum; and (ii) pharmacological treatments, such as administration of anti-inflammatory drugs, immunosuppressive agents, and anti-diarrheal drugs. However, these treatments often fall short of expectations and may lead to severe side effects and adverse reactions.

US 11690883 B2 discloses that *Lactobacillus acidophilus* TW01, which is deposited at the Leibniz Institute DSMZ-German Collection of Microorganisms and Cell Cultures GmbH under an accession number DSM 33990 in accordance with the Budapest Treaty and which is isolated from a coffee fermented broth of coffee grounds, can exhibit tolerance to acid and bile salts, and hence is capable of modulating gut immunity.

In view of the aforesaid, there is still a need to develop an effective way for alleviating colitis.

Therefore, an object of the present disclosure is to provide a composition for use in alleviating colitis, which can alleviate at least one of the drawbacks of the prior art.

According to an aspect of the disclosure, there is provided a composition for use in alleviating colitis according to claim 1.

Other features and advantages of the disclosure will become apparent in the following detailed description of the embodiment(s) with reference to the accompanying drawings. It is noted that various features may not be drawn to scale.
FIG. 1 shows the FITC-D4000 concentration determined in each group of mice in Example 1, *infra*, in which the symbol "***" represents *p*<0.001 (compared with the normal control group), and the symbol "##" represents *p*<0.01 (compared with the pathological control group).
FIG. 2 shows the IL-1β concentration of the colon tissue determined in each group of mice in Example 1, *infra*, in which the symbol "***" represents *p*<0.001 (compared with the normal control group), and the symbol "##" represents *p*<0.01 (compared with the pathological control group).

For the purpose of this specification, it will be clearly understood that the word "comprising" means "including but not limited to", and that the word "comprises" has a corresponding meaning.

It is to be understood that, if any prior art publication is referred to herein, such reference does not constitute an admission that the publication forms a part of the common general knowledge in the art, in Taiwan or any other country.

Unless otherwise defined, all technical and scientific terms used herein have the meaning commonly understood by a person skilled in the art to which the present disclosure belongs. One skilled in the art will recognize many methods and materials similar or equivalent to those described herein, which could be used in the practice of the present disclosure. Indeed, the present disclosure is in no way limited to the methods and materials described.

The present disclosure provides a composition for use in alleviating colitis, which includes *Lactobacillus acidophilus* TW01.

The *Lactobacillus acidophilus* TW01 is deposited at the Leibniz Institute DSMZ-German Collection of Microorganisms and Cell Cultures GmbH under an accession number DSM 33990 in accordance with the Budapest Treaty.

According to the present disclosure, the composition for use in alleviating colitis may be administered to a subject in need thereof.

As used herein, the term "administered" can be used interchangeably with other term such as "administration", and means introducing, providing or delivering a pre-determined active ingredient to a subject by any suitable routes to perform its intended function.

As used herein, the term "subject" refers to any animal of interest, such as humans, monkeys, cows, sheep, horses, pigs, goats, dogs, cats, mice, and rats.

According to the present disclosure, the colitis may be selected from the group consisting of ulcerative colitis, microscopic colitis, ischemic colitis, infectious colitis, and combinations thereof. In certain embodiments, the colitis is ulcerative colitis.

According to the present disclosure, the *Lactobacillus acidophilus* TW01 may be viable or non-viable bacterial cells, concentrated or non-concentrated, a liquid, a paste, a semi-solid, or a solid (e.g., a pellet, a granule, or a powder), and may be heat-inactivated, frozen, dried, or freeze-dried (e.g., may be in freeze-dried form or spray/fluid bed dried form). In certain embodiments, the *Lactobacillus acidophilus* TW01 is present in a form of viable bacterial cells.

According to the present disclosure, the *Lactobacillus acidophilus* TW01 may be prepared as a bacterial suspension having a total bacterial concentration ranging from 10⁴ CFU/mL to 10⁹ CFU/mL. In certain embodiments, the *Lactobacillus acidophilus* TW01 may be prepared as a bacterial suspension having a total bacterial concentration ranging from 10⁷ CFU/mL to 10⁹ CFU/mL. In an exemplary embodiment, the *Lactobacillus acidophilus* TW01 may be prepared as a bacterial suspension having a total bacterial concentration of 10⁷ CFU/mL.

According to the present disclosure, the composition may be formulated as a food product using a standard technique well known to one of ordinary skill in the art. For example, the composition may be formulated in the form of a food additive, which is added to an edible material to prepare a food product for human or animal consumption.

As used herein, the term "food product" refers to any article or substance that can be ingested by a subject into the body thereof. Examples of the food product may include, but are not limited to, milk powders, fermented milk, yogurt, butter, beverages (e.g., tea, coffee, etc.), functional beverages, a flour product, baked foods, confectionery, candies, fermented foods, animal feeds, health foods, and dietary supplements.

According to the present disclosure, the food product may further include a food additive widely employed in the art of food-manufacturing. Examples of the food additive may include, but are not limited to, a starch, a dextrin, lactose, maize flours, rice flours, tricalcium phosphate, silicon dioxide, magnesium stearate, calcium carbonate, sucrose, glucose, fructose, a sugar alcohol, an oligosaccharide, a sugar substitute, fruit juice powders, yeast powders, skim milk powders, casein, whey protein, soybean protein, amino acid, citric acid, citrate, lactic acid, lactate and nucleotide.

According to the present disclosure, the composition may be prepared in the form of a pharmaceutical composition. The pharmaceutical composition may be formulated into a dosage form suitable for oral administration using technology well known to those skilled in the art.

According to the present disclosure, examples of the dosage form suitable for oral administration may include, but are not limited to, sterile powders, tablets, troches, lozenges, pellets, capsules, dispersible powders or granules, solutions, suspensions, emulsions, syrup, elixir, slurry, and the like.

According to the present disclosure, the pharmaceutical composition may further include a pharmaceutically acceptable carrier widely employed in the art of drug-manufacturing. For instance, the pharmaceutically acceptable carrier may include one or more of the following agents: solvents, buffers, emulsifiers, suspending agents, decomposers, disintegrating agents, dispersing agents, binding agents, excipients, stabilizing agents, chelating agents, diluents, gelling agents, preservatives, wetting agents, lubricants, absorption delaying agents, liposomes, and the like. The choice and amount of the aforesaid agents are within the expertise and routine skills of those skilled in the art.

According to the present disclosure, the dose and frequency of administration of the composition including *Lactobacillus acidophilus* TW01 may vary depending on the following factors: the severity of the illness or disorder to be treated, routes of administration, and age, physical condition and response of the subject to be treated. In general, *Lactobacillus acidophilus* TW01 may be administered in a single dose or in several doses.

The disclosure will be further described by way of the following examples. However, it should be understood that the following examples are solely intended for the purpose of illustration and should not be construed as limiting the disclosure in practice.

### EXAMPLES

### General Experimental Materials

### 1. Lactobacillus acidophilus TW01

*Lactobacillus acidophilus* TW01, which is known and readily available to the public and which is disclosed in TW I788840 B and US 11690883 B2, has been deposited at the Bioresource Collection and Research Center (BCRC) of the Food Industry Research and Development Institute (FIRDI) (No. 331, Shih-Pin Rd., Hsinchu City 300, Taiwan). In addition, *Lactobacillus acidophilus* TW01 has also been deposited under the terms of the Budapest Treaty on the International Recognition of the Deposit of Microorganisms for the Purpose of Patent Procedure at the International Depositary Authority, i.e., Leibniz Institute DSMZ-German Collection of Microorganisms and Cell Cultures GmbH (Inhoffenstr. 7B, D-38124 Braunschweig, Germany) in accordance with the Budapest Treaty.

The relevant information regarding *Lactobacillus acidophilus* TW01 (including accession number and date of deposit) is listed in Table 1 below.

**Table 1**

| LAB strain | Accession number | Date of deposit |
|---|---|---|
| *Lactobacillus acidophilus* TW01 | BCRC 911039 | March 05, 2021 |
| | DSM 33990 | August 02, 2021 |

### 2. Preparation of bacterial suspension of Lactobacillus acidophilus TW01

The *Lactobacillus acidophilus* TW01 described in section 1 of "General Experimental Materials" was inoculated in a Lactobacilli MRS broth (manufacturer: Difco Laboratories Inc., U.S.A), followed by cultivation in an incubator (37°C) under an anaerobic condition for 24 hours, so as to obtain an inoculum. Thereafter, the inoculum was subjected to a centrifugation treatment at 3,000 rpm for 20 minutes, followed by removing the resultant cell culture supernatant, so as to collect the resultant cell pellet. Subsequently, the collected cell pellet was washed with an appropriate amount of sodium chloride salt solution 0.85 % two times. Afterwards, an appropriate amount of phosphate-buffered saline (PBS) was added to suspend the washed cell pellet and to adjust the bacterial concentration to the desired bacterial concentration which was determined using a plate counting medium, thereby obtaining a bacterial suspension having a bacterial concentration of 10⁷ CFU/mL (abbreviated as TW01-bacterial suspension).

### 3. Experimental mice

Male C57BL/6 mice (6 weeks to 8 weeks old, with a body weight of approximately 20 g) used in the following experiments were purchased from the National Laboratory Animal Center of the National Applied Research Laboratories. Each of the experimental mice was housed in an animal room under the following laboratory conditions: an alternating 12-hour light and 12-hour dark cycle, a temperature maintained from 21 ° C to 25 °C, and a relative humidity maintained from 60% to 70%. The experimental mice were provided with water and fed *ad libitum.* All experimental procedures involving the experimental mice were in compliance with the legal provision of the Institutional Animal Care and Use Committee of the National Taiwan University, and were carried out according to the Guideline for the Care and Use of Laboratory Animals.

### General Procedures:

### 1. Statistical analysis

All the experiments described below were performed in triplicates. The experimental data of all the test groups are expressed as mean ± standard deviation (SD), and were analyzed using one-way analysis of variance (one-way ANOVA) followed by Tukey's test, so as to evaluate the differences between the groups. Statistical significance is indicated by p<0.05.

### Example 1. Evaluation of the effect of Lactobacillus acidophilus TW01 on alleviating colitis

In this example, the applicant used dextran sulfate sodium (DSS) to induce colitis in male C57BL/6 mice, followed by conducting analysis of intestinal permeability, histopathologic analysis of colon tissue, and determination of IL-1β concentration in colon tissue, so as to evaluate the effect of *Lactobacillus acidophilus* TW01 on alleviating colitis, generally with reference to the method described in Chassaing B. et al. (2014), Curr. Protoc. Immunol., 104:15.25.1-15.25.14.

### Experimental Procedures:

### A. Administration of TW01-bacterial suspension and induction of colitis

First, the male C57BL/6 mice as described in section 3 of "General Experimental Materials" were randomly divided into 3 groups (n=10 mice in each group), including a normal control group, a pathological control group, and an experimental group. Next, the mice in the experimental group were fed, via oral gavage, with 0.2 mL of the TW01-bacterial suspension prepared as described in section 2 of "General Experimental Materials", and the mice in each of the normal control group and the pathological control group were fed, via oral gavage, with 0.2 mL of phosphate-buffered saline (PBS). Each mouse was fed once daily for a total period of 14 days.

Starting on the 8^{th} day, before each daily administration of the TW01-bacterial suspension or the PBS, drinking water of mice in the pathological control group and the experimental group was replaced with drinking water containing 2.5% DSS (manufacturer: MP Biomedicals; Cat. no.: 160110), and this treatment (i.e., the use of the DSS to induce colitis) was continued until the end of the 14-day experimental period, so as to induce colitis. In addition, each mouse in the normal control group received no treatment.

### B. Analysis of intestinal permeability

After completing the 14^{th} day of administration of the TW01-bacterial suspension or the PBS, the mice in each group were fed, via oral gavage, with 200 µL of PBS containing 60 mg/mL of fluorescein isothiocyanate-dextran 4000 (abbreviated as FiTC-D4000, manufacturer: Sigma-Aldrich). At the 4^{th} hour after administration of the FITC-D4000, blood samples were collected from facial veins of the mice of each group via puncture, followed by subjecting the thus collected blood samples to a centrifugation treatment at 4,600 rpm and 4°C for 5 minutes, so as to collect the resultant supernatants. Subsequently, each of the collected supernatants was diluted 10-fold with double distilled water, so as to obtain a serum sample. Thereafter, the serum sample was subjected to determination of FITC-D4000 concentration using a microplate spectrophotometer (manufacturer: BioTek Instruments, model no.: Epoch) with an excitation wavelength of 485 nm and an emission wavelength of 528 nm in accordance with the manufacturer's instructions. The thus obtained fluorescence intensities of the FITC-D4000 for the mice in each group were converted to FITC-D4000 concentrations thereof that were expressed in ng/mL according to a standard curve prepared in advance using standards with known FITC-D4000 concentrations (i.e., 0 ng/mL, 100 ng/mL, 250 ng/mL, 500 ng/mL, 1000 ng/mL, 1500 ng/mL, 2500 ng/mL, and 3000 ng/mL) relative to their corresponding fluorescence intensities.

The data thus obtained were analyzed according to the procedures as described in section 1 of "General Procedures".

### C. Histopathologic analysis of colon tissue

After completing the aforesaid experiments in section B of "Experimental Procedures" of this example, the mice in each group were anesthetized with an anesthetic, followed by sacrificing the mice, and then colon tissue was obtained from each mouse carcass. Next, a part of the colon tissue was subjected to a fixation treatment with a 10% formalin at room temperature for 24 hours, and then the thus fixed colon tissue was embedded in paraffin, followed by conducting a slicing process, so as to obtain a tissue section with a thickness of 3 µm.

Thereafter, the tissue section was subjected to hematoxylin-eosin staining using a staining protocol well-known to those skilled in the art, and then was photographed using an optical microscope (manufacturer: Olympus; Model no.: BX53) at a magnification of 40×, 100×, and 400×. Afterwards, 5 types of histopathological changes in colitis of the tissue section, as listed in Table 2 below, was assessed by a veterinary pathologist from the National Chung Hsing University, Taichung, Taiwan, according to the methods described in Shackelford C. et al. (2002), Toxicol. Pathol., 30:93-96 and Huang Y.T., et al. (2013), PLoS One, 8(5): e64152.

**Table 2**

| |
|---|
| 5 types of histopathological changes in colitis |
| Crypt loss |
| Crypt regeneration |
| Submucosal edema |
| Monocyte-driven inflammation |
| Ulcer with fibroblast infiltration |

Each type of histopathological change in colitis was graded by histopathological score on a scale of 0 to 5, with higher scores indicating greater severity. The total score for the 5 types of histopathological changes in colitis was calculated, followed by determining an average histopathological score.

The data thus obtained were analyzed according to the procedures as described in section 1 of "General Procedures". The results were shown in Table 3 below.

### D. Determination of IL-1β concentration in colon tissue

First, 0.5 g of the colon tissue of mouse in each group prepared as described in section C of "Experimental Procedures" of this example was collected, followed by adding appropriate amounts of 1.0 mm grinding beads and sterile PBS containing protease inhibitor cocktail EDTA free (manufacturer: Abcam, Cat. no.: ab270055), and then a homogenization treatment was conducted using a grinder, so as to obtain a homogeneous mixture.

Next, the homogeneous mixture was subjected to a centrifugation treatment at 12,000 rpm and 4°C for 10 minutes, so as to collect the resultant supernatant. The collected supernatant was then subjected to determination of IL-1β concentration using a mouse IL-1β ELISA Kit (manufacturer: R&D Systems, Cat. no.: DY401) in accordance with the manufacturer's instructions.

The data thus obtained were analyzed according to the procedures as described in section 1 of "General Procedures".

### Results:

### A. Analysis of intestinal permeability

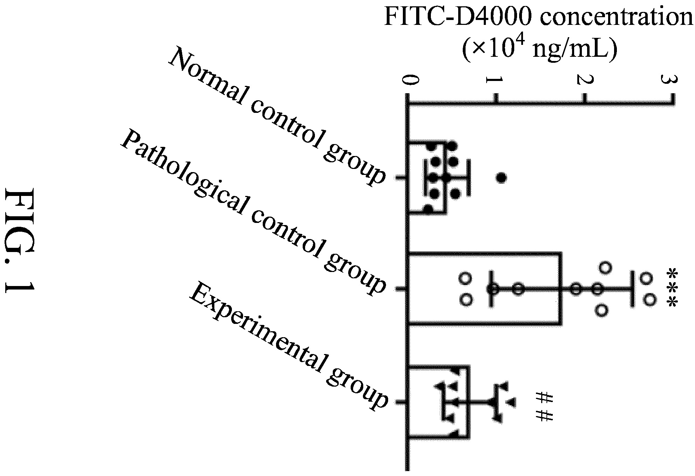

FIG. 1 shows the FITC-D4000 concentration determined in each group of mice. As shown in FIG. 1, compared with the normal control group, the FITC-D4000 concentration determined in the pathological control group showed a significant increase, indicating that the DSS successfully induced colitis in mice by destroying intestinal barrier function, thereby increasing intestinal permeability (i.e., leaky gut). In addition, compared with the pathological control group, the FITC-D4000 concentration determined in the experimental group showed a significant decrease. These results demonstrate that *Lactobacillus acidophilus* TW01 can effectively improve intestinal barrier function.

### B. Histopathologic analysis of colon tissue

**Table 3**

| Group | Average histopathological score |
|---|---|
| Normal control group | 0 |
| Pathological control group | 1.5±1.3* |
| Experimental group | 0.7±0.7*# |

| | |
|---|---|
| Note: The symbol "*" represents *p*<0.05 (compared with the normal control group), and the symbol "#" represents *p*<0.05 (compared with the pathological control group). | |

Table 3 shows the average histopathological score of colon tissue determined in each group of mice. As shown in Table 3, compared with the normal control group, the average histopathological score determined in the pathological control group showed a significant increase, indicating that the DSS successfully induced colitis in mice. In addition, compared with the pathological control group, the average histopathological score determined in the experimental group showed a significant decrease. These results demonstrate that *Lactobacillus acidophilus* TW01 can effectively alleviate colitis-associated lesions in colon tissue.

### C. Determination of IL-1β concentration in colon tissue

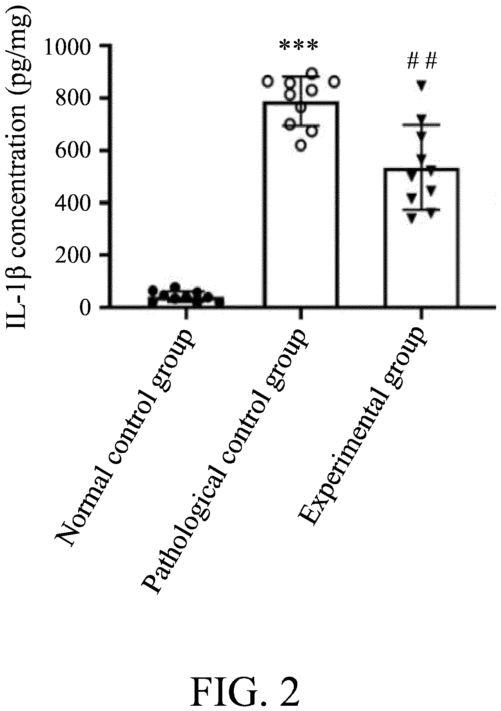

FIG. 2 shows the IL-1β concentration of the colon tissue determined in each group of mice. As shown in FIG. 2, compared with the normal control group, the IL-1β concentration determined in the pathological control group showed a significant increase, indicating that the DSS successfully induced colitis in mice. In addition, compared with the pathological control group, the IL-1β concentration determined in experimental group showed a significant decrease. These results demonstrate that *Lactobacillus acidophilus* TW01 can effectively reduce inflammation in colon tissue.

Summarizing the above test results, it is clear that *Lactobacillus acidophilus* TW01 of the present disclosure is capable of effectively improving intestinal barrier function, alleviating colitis-associated lesions in colon tissue, and reducing inflammation in colon tissue, and hence has a potential to be used in the preparation of a composition for alleviating colitis.

In the description above, for the purposes of explanation, numerous specific details have been set forth in order to provide a thorough understanding of the embodiment(s). It will be apparent, however, to one skilled in the art, that one or more other embodiments may be practiced without some of these specific details. It should also be appreciated that reference throughout this specification to "one embodiment," "an embodiment," an embodiment with an indication of an ordinal number and so forth means that a particular feature, structure, or characteristic may be included in the practice of the disclosure. It should be further appreciated that in the description, various features are sometimes grouped together in a single embodiment, figure, or description thereof for the purpose of streamlining the disclosure and aiding in the understanding of various inventive aspects; such does not mean that every one of these features needs to be practiced with the presence of all the other features. In other words, in any described embodiment, when implementation of one or more features or specific details does not affect implementation of another one or more features or specific details, the one or more features may be singled out and practiced alone without the another one or more features or specific details. It should be further noted that one or more features or specific details from one embodiment may be practiced together with one or more features or specific details from another embodiment, where appropriate, in the practice of the disclosure.

## Claims

1. A composition for use in alleviating colitis,
wherein the composition comprises *Lactobacillus acidophilus* TW01 which is deposited at the Leibniz Institute DSMZ-German Collection of Microorganisms and Cell Cultures GmbH under an accession number DSM 33990 in accordance with the Budapest Treaty.

2. The composition for use in alleviating colitis as claimed in claim 1, wherein the *Lactobacillus acidophilus* TW01 is present in a form of viable or non-viable bacterial cells.

3. The composition for use in alleviating colitis as claimed in claim 1 or 2, wherein the composition is formulated as a food product or a pharmaceutical composition.

4. The composition for use in alleviating colitis as claimed in claim 3, wherein the pharmaceutical composition is in an oral dosage form.
